# EUROPÄISCHE PATENTSCHRIFT

(11) **EP 4 125 722 B1**
(45) Veröffentlichungstag und Bekanntmachung des Hinweises auf die Patenterteilung: **03.01.2024**
(21) Anmeldenummer: 21711544.3
(22) Anmeldetag: 11.03.2021
(51) Int. Cl.: A61F 2/18

(54) **LÄNGENEINSTELLBARE GEHÖRKNÖCHELCHENPROTHESE**
LENGTH-ADJUSTABLE OSSICLE PROSTHESIS
PROTHÈSE D'OSSELETS RÉGLABLE EN LONGUEUR

(30) Priorität: 31.03.2020 DE 102020108887
(43) Veröffentlichungstag der Anmeldung: 08.02.2023
(73) Patentinhaber: MED-EL Elektromedizinische Geräte GmbH, 6020 Innsbruck (AT)
(72) Erfinder: STEINHARDT, Uwe, 72145 Hirrlingen (DE); HAUSCH, Jörg, 72131 Ofterdingen (DE); KUEN, Clemens, 6460 Imst (AT); MAIR, Lukas, 6130 Schwaz (AT)
(74) Vertreter: Witte, Weller & Partner Patentanwälte mbB
(86) Internationale Anmeldenummer: PCT/EP2021/056148
(87) Internationale Veröffentlichungsnummer: WO 2021/197789

(56) Entgegenhaltungen:
- EP-A1- 2 103 282
- EP-A2- 0 998 884
- WO-A1-2018/052866
- DE-B3-102017 115 806

## Beschreibung

Die Erfindung betrifft eine längeneinstellbare Gehörknöchelchenprothese mit den Merkmalen des Oberbegriffs des Anspruchs 1.

Gehörknöchelchenprothesen dienen als Ersatz eines oder mehrerer Gehörknöchelchen im menschlichen Mittelohr und übertragen anstatt des oder der ersetzten Gehörknöchelchen durch Schallwellen erzeugte Schwingungen am Trommelfell auf das ovale Fenster des Innenohrs. Die Länge einer Gehörknöchelchenprothese muss individuell an den zu überbrückenden Abstand vom Trommelfell an den anzukoppelnden Gehörknöchelchen, beispielsweise dem Steigbügel, oder dem ovalen Fenster des jeweiligen Patienten angepasst werden.

Die Patentanmeldung EP 0 998 884 A2 offenbart eine längeneinstellbare Gehörknöchelchenprothese mit einem Kopfelement, das innen an einem Trommelfell angeordnet wird, einem Fußelement das am Steigbügel befestigt wird, und einem stiftförmigen Schaft der das Kopfelement und das Fußelement verbindet. Das Kopfelement weist einen ovalen Ring auf, von dem Arme nach innen zu einer Art "Nabe" führen, welche ein Loch aufweist, in das ein Schlitz mündet. Der Schaft der bekannten Gehörknöchelchenprothese geht durch das Loch in der "Nabe" des Kopfelements hindurch, so dass das Kopfelement auf dem Schaft verschiebbar und dadurch ein Abstand des Kopfelements vom Fußelement der Gehörknöchelchenprothese einstellbar ist. Durch Zusammendrücken des Schlitzes kann das Loch verengt und das Kopfelement auf dem Schaft festgeklemmt werden. Der Abstand des Kopfelements vom Fußelement ist die Länge der Gehörknöchelchenprothese, der Schaft der Gehörknöchelchenprothese muss auf diese Länge abgelängt werden.

Das Patent DE 10 2017 115 806 B3 offenbart ein ebenfalls ringförmiges Kopfelement für eine Gehörknöchelchenprothese, das auf dem Schaft einer Gehörknöchelchenprothese verschiebbar und festklemmenbar ist. Ein Ring des Kopfelements weist in seinem Innern einen Ω-förmigen Arm auf, dessen Füße an einander gegenüberliegenden Umfangsstellen einstückig in den Ring übergehen. In einer Mitte weist der Ω-förmige Arm ein Loch für den Schaft der Gehörknöchelchenprothese auf, das von einem Schlitz durchsetzt ist, der auf einer konvexen Außenseite des Ω-förmigen Arms offen ist. Durch elastisches Zusammendrücken des Rings an den Füßen des Ω-förmigen Arms lässt sich das Loch aufweiten, so dass das Kopfelement auf dem Schaft verschiebbar ist. Ist der Ring entspannt, klemmt sein Loch auf dem Schaft fest. Nachteilig an dieser Anordnung ist, dass der Schaft nur verschiebbar ist, solange der Ring elastisch zusammengedrückt wird. Dies erschwert die Handhabung für den Chirurgen zur Längeneinstellung der Gehörknöchelchenprothese enorm.

Aufgabe der Erfindung ist eine Gehörknöchelchenprothese vorzuschlagen, bei der ein Abstand eines Kopfelements von einem Fußelement einfach einstellbar ist. Der Abstand des Kopfelements vom Fußelement wird als Länge der Gehörknöchelchenprothese angesehen.

Diese Aufgabe wird durch die Merkmale des Anspruchs 1 gelöst. Die abhängigen Ansprüche haben Weiterbildungen und vorteilhafte Ausgestaltungen der Erfindung zum Gegenstand.

Die erfindungsgemäße Gehörknöchelchenprothese weist ein Kopfelement, ein Fußelement und ein Verbindungselement, das das Kopfelement mit dem Fußelement verbindet, auf. Das Kopfelement ist zu einer Anordnung an einer Innenseite eines Trommelfells und das Fußelement zur Anordnung an einem Gehörknöchelchen oder ovalen Fenster des menschlichen Mittelohrs, insbesondere zur Befestigung am Steigbügel, vorgesehen. Als Trommelfell ist in diesem Zusammenhang sowohl ein vorhandenes, teilweise vorhandenes als auch ein künstlich vollständiges oder weilweise rekonstruiertes Trommelfell sowie ein teilweise vorhandenes und der restliche Teil rekonstruiertes Trommelfell zu verstehen. Die Rekonstruktion umfasst jegliche Form der Herstellung einer schwingfähigen Membran, beispielsweise mit Körpergewebe oder einer künstlich angelegten Schwingungsmembran zur mechanischen Schallübertragung. Mitumfasst ist eine Anordnung oder Befestigung des Kopfelements an einem Gehörknöchelchen und des Fußelements an einem anderen Gehörknöchelchen als dem Steigbügel oder an anderer Stelle in einem menschlichen Ohr. Als Verbindungselement weist die Gehörknöchelchenprothese insbesondere einen Stift auf.

Das Kopfelement ist erfindungsgemäß als Klemme ausgeführt oder weist eine Klemme auf, die in einer offenen oder entspannten Stellung in einer Längsrichtung des Verbindungselements verschiebbar und in einer gespannten Stellung am Verbindungselement festgeklemmt ist, so dass ein Abstand des Kopfelements vom Fußelement einstellbar und das Kopfelement durch Klemmen in dem eingestellten Abstand an dem Verbindungselement festlegbar ist.

Das Kopfelement beziehungsweise die Klemme weist zwei oder mehr Führungs- und Klemmelemente auf, die das Kopfelement bei offener Klemme in der Längsrichtung des Verbindungselements verschiebbar an dem Verbindungselement führen und die bei gespannter Klemme das Kopfelement an dem Verbindungselement festklemmen. Dabei soll eine Klemmkraft so groß sein, dass sich das Kopfelement bei gespannter Klemme bei in einem menschlichen Ohr auftretenden Belastungen weder gegenüber dem Verbindungselement dreht, noch verschiebt noch in sonstiger Weise gegenüber dem Verbindungselement bewegt. Andererseits soll die Klemmkraft nicht so groß sein, dass sich das Kopfelement über den langen implantierten Zeitraum hinweg quer zur Axialebene des Rings des Kopfelements oder allgemein quer zu seiner Fläche verformt.

Des Weiteren weist das Kopfelement der erfindungsgemäßen Gehörknöchelchenprothese vorzugsweise für jedes Führungs- und Klemmelement ein Spannelement auf, das beispielsweise insbesondere hinsichtlich seiner Funktion einem Griff oder einem Hebel einer Zange vergleichbar ist. Mit den Spannelementen sind die Führungs- und Klemmelemente so gegen das Verbindungselement spannbar, dass sie an dem Verbindungselement festklemmen und auf diese Weise das Kopfelement durch Klemmen an dem Verbindungselement festlegen. Vorzugsweise sind die Spannelemente in der Spannrichtung elastisch, um eine Spannkraft beziehungsweise Klemmkraft aufzubringen.

Um das Kopfelement beziehungsweise die Klemme in der gespannten Stellung zu halten und dadurch das Kopfelement gegen Verschieben und Drehen an dem Verbindungselement festzulegen, weist das Kopfelement eine Verriegelung auf, die, wenn sie geschlossen ist, die Spannelemente in der gespannten Stellung halten, in der die Spannelemente die Führungs- und Klemmelemente gegen das Verbindungselement spannen.

Wenn die Verriegelung offen ist, lässt sich das Kopfelement der erfindungsgemä-ßen Gehörknöchelchenprothese in der Längsrichtung des Verbindungselements verschieben und dadurch der Abstand des Kopfelements vom Fußelement einstellen. Bei offener Verriegelung lässt sich das Kopfelement frei oder eventuell reibungsbehaftet in der Längsrichtung des Verbindungselements verschieben, wobei die Reibung bei offener Verriegelung niedriger als die Klemmkraft bei geschlossener Verriegelung und gespannter Klemme ist und vorzugsweise nur einen Bruchteil der Klemmkraft beträgt. Bei geschlossener Verriegelung und gespannter Klemme wird das Kopfelement mit der Klemmkraft gegen Verschieben und Drehen an dem Verbindungselement gehalten. Die Erfindung ermöglicht eine einfache Einstellung des Abstands des Kopfelements vom Fußelement und Festlegung des Kopfelements in dem eingestellten Abstand. "Längeneinstellbar" ist hier im Sinne der Einstellung des Abstands des Kopfelements vom Fußelement zu verstehen.

Zur verschiebbaren Führung des Kopfelements in der Längsrichtung des Verbindungselements sieht eine Ausgestaltung der Erfindung Vertiefungen in einander zugewandten Rändern oder Flächen der Führungs- und Klemmelemente vor, in beziehungsweise zwischen denen das Verbindungselement aufgenommen ist. Die Vertiefungen sind beispielsweise rund oder eckig, beispielsweise kreisförmig oder V-förmig, und können als Führungen und Klemmabschnitte zum verschiebbaren Führen in der Längsrichtung des Verbindungselement bei offener Klemme und zum Festklemmen an beziehungsweise auf dem Verbindungselement bei gespannter Klemme aufgefasst werden.

In bevorzugter Ausgestaltung der Erfindung weist das Kopfelement einen an einer Umfangsstelle offenen Ring auf, der dadurch in einer Axialebene des Rings durch die offene Umfangsstelle elastisch und/oder plastisch zusammendrückbar ist. Von dem Ring stehen die Führungs- und Klemmelemente nach innen ab, so dass sich ihr Abstand voneinander durch Zusammendrücken des Rings verkleinern lässt und die Führungs- und Klemmelemente gegen das Verbindungselement spannen lassen, so dass das Kopfelement durch Klemmen am Verbindungselement festgelegt ist. Mit der Verriegelung ist der Ring in der gespannten Stellung schließbar, um das Kopfelement am Verbindungselement festzulegen. Der Ring und die Führungs- und Klemmelemente bilden die Klemme des Kopfelements, wobei die Verriegelung als Bestandteil der Klemme aufgefasst werden kann. Diese Ausgestaltung ermöglicht eine einfache Herstellung des Kopfelements aus einem Stück, wobei eine Einstückigkeit ein bevorzugtes jedoch kein zwingendes Merkmal der Erfindung ist.

Eine Ausgestaltung der Erfindung sieht einen Haken und ein Gegenstück, in das der Haken zum Schließen des Rings einhängbar ist, als Verriegelung oder als Teil der Verriegelung vor. In einer Weiterbildung umfasst die Verriegelung zwei oder mehr Haken auf einer Seite der Öffnung, die entlang des Rings angeordnet hintereinander angeordnet sind, wobei jeder einzelne der Haken in das Gegenstück auf der anderen Seite der Öffnung des Rings einhängbar ist, sodass je nach eingehängtem Haken in das Gegenstück der Ring enger oder weiter ist. Dadurch lässt sich die Klemmkraft einstellen beziehungsweise ein Abstand der Führungs- und Klemmelemente an das Verbindungselement beziehungsweise an einen Durchmesser des Verbindungselements anpassen. Das Kopfelement ist dadurch für Verbindungselemente mit unterschiedlichen Durchmessern verwendbar. Die Klemmkraft lässt für einen vorgegebenen Durchmesser des Verbindungselements optimal einstellen. Dabei soll eine Klemmkraft so groß sein, dass sich das Kopfelement bei gespannter Klemme bei in einem menschlichen Ohr auftretenden Belastungen weder gegenüber dem Verbindungselement dreht noch verschiebt noch in sonstiger Weise gegenüber dem Verbindungselement bewegt. Die Klemmkraft soll aber nicht so groß sein, dass sich das Kopfelement über den langen implantierten Zeitraum hinweg quer zur Axialebene des Rings des Kopfelements verformt.

Eine Ausgestaltung der Erfindung sieht eine Querschnittsschwächung an einer Umfangsstelle oder in einem Umfangsabschnitt des Rings des Kopfelements vor. Die Querschnittsschwächung bildet eine Art Gelenk, um das gegenüberliegende Umfangsabschnitte des Rings von der Querschnittsschwächung bis zur offenen Umfangsstelle gegen einander schwenkbar sind. Die Querschnittsschwächung befindet sich insbesondere der offenen Umfangsstelle gegenüber, vorzugsweise an der Innenseite des Ringes des Kopfelements.

Eine bevorzugte Ausgestaltung der Erfindung sieht Ansatzflächen zum Ansetzen eines Schließwerkzeugs zum Schließen des Rings an einer Außenseite des Rings vor. Die Ansatzflächen sind beispielsweise Vertiefungen in der Außenseite des Rings die so geformt sind, dass das Schließwerkzeug, beispielsweise eine Zange, nicht in Umfangsrichtung des Rings abrutscht, wenn es eine Schließkraft auf den Ring ausübt, das heißt Enden des Rings beiderseits der offenen Umfangsstelle zusammendrückt.

Um eine Kraftübersetzung zu erzielen, sieht eine Ausgestaltung der Erfindung vor, dass die Führungs- und Klemmelemente einen größeren Abstand von der Öffnung des Rings als von einer der Öffnung gegenüberliegenden Umfangsstelle aufweisen. Eine auf die Enden oder nahe an den Enden des Rings beiderseits der Öffnung ausgeübte Schließkraft wird entsprechend der Hebelarme in eine größere Klemmkraft übersetzt. Die Hebelarme sind die Abstände der Angriffsstellen der Schließkraft von der der Öffnung gegenüberliegenden Umfangsstelle des Rings des Kopfelements der Gehörknöchelchenprothese einerseits und die Abstände der Führungs- und Klemmelemente beziehungsweise der Führungen und Klemmabschnitte von der der Öffnung gegenüberliegenden Umfangsstelle des Rings andererseits.

Sämtliche in der Beschreibung genannte und/oder der Zeichnung dargestellte Merkmale können einzeln für sich oder in jeder beliebigen Kombination bei Ausführungen der Erfindung verwirklicht sein. Ausführungen der Erfindung, die nicht alle, sondern nur einen Teil der Merkmale eines Anspruchs, auch des unabhängigen Anspruchs, aufweisen, sind möglich.

Die Erfindung wird nachfolgend anhand eines in der Zeichnung dargestellten Ausführungsbeispiels näher erläutert. Es zeigen:
- Figur 1: eine erfindungsgemäße Gehörknöchelchenprothese in perspektivischer Darstellung; und
- Figur 2: ein Kopfelement der Gehörknöchelchenprothese in Ansicht.

Die in der Zeichnung dargestellte, erfindungsgemäße Gehörknöchelchenprothese 1 weist ein Kopfelement 2, ein Fußelement 3 und ein Verbindungselement 4 auf, das das Kopfelement 2 und das Fußelement 4 verbindet. Die Gehörknöchelchenprothese 1 ist zum Ersatz eines oder mehrerer Gehörknöchelchen in einem menschlichen Ohr vorgesehen. Dabei ist das Kopfelement 2 zu einer Anordnung an einer Innenseite eines Trommelfells des Ohrs und das Fußelement 3 zu einer Anbringung an einem Gehörknöchelchen, insbesondere an einem Steigbügel oder einem ovalen Fenster im Mittelohr eines Menschen vorgesehen. Die Gehörknöchelchenprothese 1 überträgt durch Schallwellen erzeugte Schwingungen des Trommelfells auf den Steigbügel oder das Element des Ohrs, an dem das Fußelement 3 angebracht ist. Als Trommelfell ist in diesem Zusammenhang sowohl ein vorhandenes, teilweise vorhandenes als auch ein künstlich vollständiges oder teilweise rekonstruiertes Trommelfell sowie ein teilweise vorhandenes und der restliche Teil rekonstruiert zu verstehen. Die Rekonstruktion umfasst jegliche Form der Herstellung einer schwingfähigen Membran, beispielsweise mit Körpergewebe oder einer künstlich angelegten Schwingungsmembran zur mechanischen Schallübertragung.

Das Kopfelement 2 der Gehörknöchelchenprothese 1 ist erfindungsgemäß als Klemme 15 ausgebildet. Es weist einen - im Ausführungsbeispiel - in etwa elliptischen Ring 5 auf, der an einer Umfangsstelle offen ist und der an einer gegenüberliegenden Umfangsstelle oder einem gegenüberliegenden Umfangsabschnitt dünner als im übrigen ist, das heißt eine Querschnittsschwächung 6 aufweist. Im Ausführungsbeispiel befinden sich die offene Umfangsstelle, die hier als Öffnung 7 des Rings 5 bezeichnet wird, und die Querschnittsschwächung 6 einander gegenüber an Scheiteln der Ellipse.

An einander bezüglich einer Hauptachse der Ellipse gegenüberliegenden Umfangsstellen stehen zwei Führungs- und Klemmelemente 8 vom Ring 5 nach innen, die an ihren einander zugewandten Rändern kreisbogenförmigen Vertiefungen aufweisen, die sich auf einem gedachten Kreis befinden. Die Vertiefungen bilden Führungen und Klemmabschnitte 9 für das Verbindungselement 4. Die Vertiefungen können auch eine andere Form aufweisen, beispielsweise eine V-Form. Wesentlich ist, dass die Führungen und Klemmabschnitte 9 das Kopfelement 2 in einer offenen Stellung in einer Längsrichtung des Verbindungselements 4 verschiebbar an dem Verbindungselement 4 führen und in einer geschlossenen beziehungsweise gespannten Stellung das Kopfelement 2 durch Klemmen gegen Verschieben und Drehen an dem Verbindungselement 4 festlegen. Ein Festlegen gegen Drehen ist auch durch einen Formschluss denkbar mit einem Verbindungselement 4, welches einen nicht kreisförmigen Querschnitt aufweist, auf dem das Kopfelement nicht drehbar ist (nicht dargestellt). Eine Klemmkraft des geschlossenen beziehungsweise gespannten Kopfelements 2 ist so groß, dass sich das Kopfelement 2 bei im menschlichen Ohr auftretenden Belastungen nicht gegenüber dem Verbindungselement 4 bewegt. Die Klemmkraft ist aber nicht so groß, dass sich das Kopfelement 2 über den langen implantierten Zeitraum hinweg quer zur Axialebene des Rings 5 des Kopfelements 2 verformt. Ein Verschieben oder Verdrehen des Kopfelements 2 mit einer größeren Kraft ist nicht ausgeschlossen.

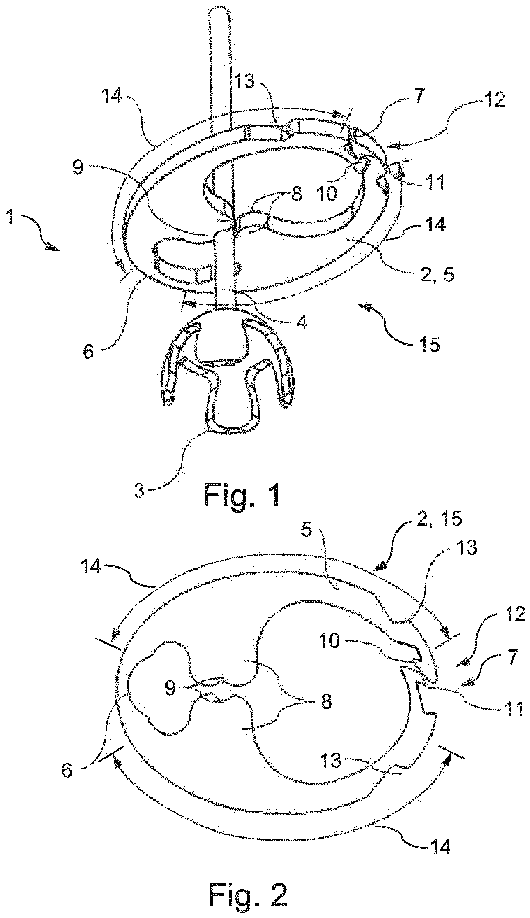

Auf einer Seite der Öffnung 7 weist der Ring 5 einen Haken 10 an seiner Außenseite und auf einer anderen Seite der Öffnung 7 einen Haken an seiner Innenseite auf. Der Haken an der Innenseite des Rings 5 bildet ein Gegenstück 11 für den Haken 10. Der Haken 10 und sein Gegenstück 11 bilden eine Verriegelung 12 und sind zum Schließen des Rings 5 beziehungsweise des als Klemme 15 ausgebildeten Kopfelements 2 der erfindungsgemäßen Gehörknöchelchenprothese 1 ineinander einhängbar, wie es Figur 1 zeigt. Figur 2 zeigt das Kopfelement 2 mit offener Verriegelung 12, offenem Ring 2 beziehungsweise offener Klemme 15 mit nicht in das Gegenstück 11 eingehängtem Haken 10.

Mit etwas Abstand in Umfangsrichtung von dem Haken 10 und dem Gegenstück 11 weist der Ring 10 des Kopfelements 2 Ausnehmungen an seiner Außenseite auf, durch die Ansatzflächen 13 zum Ansetzen eines nicht dargestellten Schließwerkzeugs gebildet sind. Die Ansatzflächen 13 verlaufen parallel zueinander und parallel zur Hauptachse des ellipsenförmigen Rings 5 oder die Ansatzflächen 13 verlaufen in Richtung der Öffnung 7 etwas auseinander, so dass ein an den Ansatzflächen 13 angesetztes Schließwerkzeug nicht in der Umfangsrichtung des Rings 5 abrutscht.

Zum Schließen des Rings 5 und damit der Klemme 15 des Kopfelements 2 werden mit beispielsweise einer nicht dargestellten Zange als Schließwerkzeug, das an den Ansatzflächen 13 angesetzt wird, die Ansatzflächen 13 einander genähert und dadurch der Haken 10 dem Gegenstück 11 der Verriegelung 12 genähert, bis der Haken 10 im Gegenstück 11 einhängt, wodurch der Ring 5 geschlossen ist. Ein Schließen des Rings 5 in anderer Weise ist nicht ausgeschlossen.

Beim Schließen des Rings 5 bildet die Querschnittsschwächung 6 eine Art Scharnier, durch die bogenförmige Abschnitte des Rings 5 zwischen der Querschnittsschwächung 6 und der Öffnung 7 schwenkbar verbunden sind. Die bogenförmige Abschnitte des Rings 5 zwischen der Querschnittsschwächung 6 und der Öffnung 7 können auch als Arme, Spannarme oder allgemein als Spannelemente 14 der Klemme 15 aufgefasst werden. Im Ausführungsbeispiel sind sie elastisch.

Beim Schließen des Rings 5 bewegen sich die beiden Führungs- und Klemmelemente 8 und die Führungen und Klemmabschnitte 9 aufeinander zu und/oder werden gegen das Verbindungselement 4 gedrückt, was auch als Spannen des Rings 5, des Kopfelements 2 beziehungsweise der Klemme 15 aufgefasst werden kann. Die Führungs- und Klemmelement 8 und die Führungen und Klemmabschnitte 9 befinden sich zwischen der das Scharnier bildenden Querschnittsschwächung 6 und den Ansatzflächen 13 für das Schließwerkzeug und vorzugsweise - wie im Ausführungsbeispiel - näher an der Querschnittsschwächung 6, wodurch sich eine Kraftübersetzung ergibt: die Schließkraft zum Zusammendrücken der Ansatzflächen 13 ist kleiner als eine Klemmkraft, mit der die Führungen und Klemmabschnitte 9 gegen das Verbindungselement 4 gedrückt, das heißt gespannt werden.

Im Ausführungsbeispiel ist das Kopfelement 2 in einem Stück durch Laserschneiden aus einem Metallblech hergestellt. Andere Herstellungsmöglichkeiten sind nicht ausgeschlossen, beispielsweise auch eine Herstellung aus mehreren, beispielsweise verschweißten Stücken und/oder eine Herstellung aus Draht.

Das Verbindungselement 4 ist im dargestellten und beschriebenen Ausführungsbeispiel der Erfindung ein Zylinderstift, der zwischen den Führungen und Klemmabschnitten 9 der Führungs- und Klemmelemente 8 des Kopfelements 2 hindurch geht und an dessen einem Ende das Fußelement 3 befestigt ist, das im Ausführungsbeispiel eine Glockenform aufweist, wobei andere Formen möglich sind. Das Verbindungselement 4 muss nicht zwingend ein Zylinderstift sein.

Wenn der Ring 5 beziehungsweise das als Klemme 15 ausgebildete Kopfelement 2 offen ist, das heißt der Haken 10 nicht in das Gegenstück 11 eingehängt ist, lässt sich das Kopfelement 2 reibungsfrei oder reibungsbehaftet in der Längsrichtung des Verbindungselements 4 auf diesem Verschieben und dadurch ein Abstand des Kopfelements 2 vom Fußelement 3 einstellen. Das Einstellen des Abstands des Kopfelements 2 vom Fußelement 3 kann auch als Längeneinstellung der Gehörknöchelchenprothese 1 aufgefasst werden. Durch Schließen der Verriegelung 12, des Rings 5 beziehungsweise des als Klemme 15 ausgebildeten Kopfelements 2 werden die Führungen und Klemmabschnitte 9 der Führungs- und Klemmelemente 8 gegen das Verbindungselement 4 gedrückt beziehungsweise gespannt und halten das Kopfelement 2 verschiebefest und drehfest auf dem Verbindungselement 4. Das Schließen kann auch als Spannen und die geschlossene Stellung des Rings 5 beziehungsweise der Klemme 15 als gespannte Stellung aufgefasst werden.

Zum Abschluss wird das Verbindungselement 4 auf einer dem Fußelement 3 abgewandten Seite des Kopfelements 2 abgetrennt, so dass das Verbindungselement 4 nicht auf der dem Fußelement 3 abgewandten Seite über das Kopfelement 2 übersteht. Das Abtrennen des Verbindungselements 4 kann auch als Ablängen des Verbindungselements 4 und der Gehörknöchelchenprothese 1 aufgefasst werden.

Die Form einer Ellipse des Rings 5 ist nicht zwingend für die Erfindung, der Ring 5 kann auch eine andere, vorzugsweise gerundete, eventuell auch eckige Form aufweisen. Auch ist der Ring 5 nicht in jedem Fall zwingend für die Erfindung, notwendig ist die Ausbildung des Kopfelements 2 als Klemme 15 mit Spannarmen oder dergleichen Spannelementen 14, von denen die Führungs- und Klemmelement 8 in Richtung des Verbindungselements 4 abstehen, so dass ihre Führungen und Klemmabschnitte 9 zum Festklemmen des Kopfelements 2 am Verbindungselement 4 gegen das Verbindungselement 4 spannbar und die Spannarme oder allgemein die Spannelemente 14 das Kopfelement 2 in dieser gespannten Stellung beispielsweise mit einer Verriegelung 12 festlegbar sind.

## Patentansprüche

1. Längeneinstellbare Gehörknöchelchenprothese, mit einem Kopfelement (2), das zu einer Anordnung an einem Trommelfell vorgesehen ist, mit einem Fußelement (3), das zu einer Anordnung an einem Gehörknöchelchen oder ovalen Fenster des menschlichen Mittelohrs vorgesehen ist, und mit einem Verbindungselement (4), das das Kopfelement (2) und das Fußelement (3) verbindet, **dadurch gekennzeichnet, dass** das Kopfelement (2) eine Klemme (15) mit Führungs- und Klemmelementen (8) aufweist, die bei offener Klemme (15) das Kopfelement (2) verschiebbar an dem Verbindungselement (4) führen und die bei gespannter Klemme (15) das Kopfelement (2) an dem Verbindungselement (4) festklemmen, dass die Klemme (15) Spannelemente (14) aufweist, mit denen die Führungs- und Klemmelemente (8) gegen das Verbindungselement (4) spannbar sind, so dass sie an dem Verbindungselement (4) festklemmen, und dass die Klemme (15) eine Verriegelung (12) aufweist, die, wenn sie geschlossen ist, die Spannelemente (14) in der gespannten Stellung hält, in der die Spannelemente (14) die Führungs- und Klemmelemente (8) gegen das Verbindungselement (4) spannen.

2. Längeneinstellbare Gehörknöchelchenprothese nach Anspruch 1, **dadurch gekennzeichnet, dass** die Führungs- und Klemmelemente (8) einander zugewandte Vertiefungen als Führungen und Klemmabschnitte (9) aufweisen, in denen das Verbindungselement (4) aufgenommen ist.

3. Längeneinstellbare Gehörknöchelchenprothese nach Anspruch 1 oder 2, **dadurch gekennzeichnet, dass** das Kopfelement (2) einen an einer Umfangsstelle offenen Ring (5) aufweist, von dem die Führungs- und Klemmelemente (8) nach innen abstehen, so dass sie durch Zusammendrücken des Rings (5) gegen das Verbindungselement (4) gespannt werden, und dass der Ring (5) mit der Verriegelung (12) der gespannten Stellung schließbar ist, in der er die Führungs- und Klemmelemente (8) gegen das Verbindungselement (4) spannt.

4. Längeneinstellbare Gehörknöchelchenprothese nach Anspruch 3, **dadurch gekennzeichnet, dass** die Verriegelung (12) einen Haken (10) auf einer Seite einer Öffnung (7) des Rings (5) aufweist, der in ein Gegenstück (11) auf einer anderen Seite der Öffnung (7) des Rings (5) einhängbar ist.

5. Längeneinstellbare Gehörknöchelchenprothese nach Anspruch 4, **dadurch gekennzeichnet, dass** die Verriegelung (12) zwei oder mehr Haken (10) auf einer Seite einer Öffnung (7) entlang des Rings (5) hintereinander aufweist, wobei jeder einzelne der Haken in das Gegenstück (11) auf der anderen Seite der Öffnung (7) des Rings (5) einhängbar ist, sodass ein Umfang des Rings (5) und/oder ein Abstand der Führungs- und Klemmelemente (8) voneinander zu einer Anpassung an Verbindungselemente (4) mit verschiedenen Durchmessern einstellbar ist.

6. Längeneinstellbare Gehörknöchelchenprothese nach einem oder mehreren der Ansprüche 3 bis 5, **dadurch gekennzeichnet, dass** der Ring (5) an einer Umfangsstelle eine Querschnittsschwächung (6) aufweist um ihn zum Spannen der Führungs- und Klemmelemente (8) gegen das Verbindungselement (4) leichter zusammendrücken zu können.

7. Längeneinstellbare Gehörknöchelchenprothese nach einem oder mehreren der Ansprüche 3 bis 6, **dadurch gekennzeichnet, dass** der Ring (5) beiderseits der Öffnung (7) Ansatzflächen (13) zum Ansetzen eines Schließwerkzeugs zum Schließen des Rings (5) an seiner Außenseite aufweist, die so geformt sind, dass das angesetzte Schließwerkzeug nicht in Umfangsrichtung des Rings (5) abrutscht, wenn es eine Schließkraft auf den Ring (5) ausübt.

8. Längeneinstellbare Gehörknöchelchenprothese nach einem oder mehreren der Ansprüche 3 bis 7, **dadurch gekennzeichnet, dass** die Führungs- und Klemmelemente (8) in einem größeren Abstand von der Öffnung (7) des Rings (5) als von einer der Öffnung (7) gegenüberliegenden Umfangsstelle angeordnet sind.

9. Längeneinstellbare Gehörknöchelchenprothese nach einem oder mehreren der Ansprüche 1 bis 8, **dadurch gekennzeichnet, dass** das Fußelement (3) zu einer Anbringung an dem Steigbügel oder dem ovalen Fenster des Mittelohrs eines Menschen ausgebildet ist.

## Claims

1. A length-adjustable ossicular prosthesis, comprising a head element (2), which is intended to be disposed at an eardrum, a foot element (3), which is intended to be disposed at an ossicle or oval window of the human middle ear, and a connecting element (4), which connects the head element (2) and the foot element (3), **characterized in that** the head element (2) comprises a clip (15) including guiding and clamping elements (8), which, when the clip (15) is open, displaceably guide the head element (2) at the connecting element (4) and, when the clip (15) is tensioned, securely clamp the head element (2) to the connecting element (4); **in that** the clip (15) comprises tensioning elements (14), by way of which the guiding and clamping elements (8) can be tensioned against the connecting element (4) so as to be securely clamped to the connecting element (4); and **in that** the clip (15) comprises a locking mechanism (12) that, when closed, holds the tensioning elements (14) in the tensioned position in which the tensioning elements (14) tension the guiding and clamping elements (8) against the connecting element (4).

2. The length-adjustable ossicular prosthesis according to claim 1, **characterized in that** the guiding and clamping elements (8) comprise mutually facing depressions serving as guides and clamping sections (9) in which the connecting element (4) is accommodated.

3. The length-adjustable ossicular prosthesis according to claim 1 or 2, **characterized in that** the head element (2) comprises a ring (5) that is open at a point of the circumference, from which the guiding and clamping elements (8) project inwardly so that these are tensioned against the connecting element (4) when the ring (5) is pressed together, and that the ring (5) can be closed by way of the locking mechanism (12) in the tensioned position in which the ring tensions the guiding and clamping elements (8) against the connecting element (4).

4. The length-adjustable ossicular prosthesis according to claim 3, **characterized in that** the locking mechanism (12) comprises a hook (10) on one side of an opening (7) of the ring (5), which can engage with a mating piece (11) on another side of the opening (7) of the ring (5).

5. The length-adjustable ossicular prosthesis according to claim 4, **characterized in that** the locking mechanism (12) comprises two or more hooks (10) behind one another along the ring (5) on one side of an opening (7), each of the individual hooks being engageable with the mating piece (11) on the other side of the opening (7) of the ring (5) so that a circumference of the ring (5) and/or a distance of the guiding and clamping elements (8) with respect to one another can be adjusted for adaptation to connecting elements (4) having differing diameters.

6. A length-adjustable ossicular prosthesis according to one or more of claims 3 to 5, **characterized in that** the ring (5) has a reduced cross-sectional area (6) at a point of the circumference allowing for more easy pressing together for tensioning the guiding and the clamping elements (8) against the connecting element (4).

7. A length-adjustable ossicular prosthesis according to one or more of claims 3 to 6, **characterized in that** the ring (5), on both sides of the opening (7), comprises application surfaces (13) on the outer side thereof for applying a closing tool for closing the ring (5), which are shaped so that the applied closing tool does not slip in the circumferential direction of the ring (5) when exerting a closing force on the ring (5).

8. A length-adjustable ossicular prosthesis according to one or more of claims 3 to 7, **characterized in that** the guiding and clamping elements (8) are disposed at a greater distance with respect to the opening (7) of the ring (5) than with respect to a point of the circumference which is located opposite the opening (7).

9. A length-adjustable ossicular prosthesis according to one or more of claims 1 to 8, **characterized in that** the foot element (3) is designed to be provided at the stirrup or the oval window of the middle ear of a person.

## Revendications

1. Prothèse d'osselets réglable en longueur, avec un élément de tête (2) prévu pour être agencé sur un tympan, avec un élément de pied (3) prévu pour être agencé sur un osselet ou une fenêtre ovale de l'oreille moyenne humaine, et avec un élément de liaison (4) qui relie l'élément de tête (2) et l'élément de pied (3), **caractérisée en ce que** l'élément de tête (2) présente une pince (15) avec des éléments de guidage et de serrage (8), qui, lorsque la pince (15) est ouverte, guident l'élément de tête (2) de manière coulissante sur l'élément de liaison (4) et qui, lorsque la pince (15) est tendue, bloquent l'élément de tête (2) sur l'élément de liaison (4), **en ce que** la pince (15) présente des éléments de tension (14) avec lesquels les éléments de guidage et de serrage (8) peuvent être tendus contre l'élément de liaison (4), de telle sorte qu'ils sont bloqués sur l'élément de liaison (4), et **en ce que** la pince (15) présente un verrou (12) qui, lorsqu'il est fermé, maintient les éléments de tension (14) dans la position tendue dans laquelle les éléments de tension (14) tendent les éléments de guidage et de serrage (8) contre l'élément de liaison (4).

2. Prothèse d'osselets réglable en longueur selon la revendication 1, **caractérisée en ce que** les éléments de guidage et de serrage (8) présentent des creux tournés les uns vers les autres en tant que guides et des sections de serrage (9), dans lesquelles l'élément de liaison (4) est reçu.

3. Prothèse d'osselets réglable en longueur selon la revendication 1 ou 2, **caractérisée en ce que** l'élément de tête (2) présente une bague (5) ouverte à un emplacement périphérique, à partir de laquelle les éléments de guidage et de serrage (8) font saillie vers l'intérieur, de telle sorte qu'ils sont tendus contre l'élément de liaison (4) par compression de la bague (5), et **en ce que** la bague (5) peut être fermée avec le verrou (12) dans la position tendue, dans laquelle elle tend les éléments de guidage et de serrage (8) contre l'élément de liaison (4).

4. Prothèse d'osselets réglable en longueur selon la revendication 3, **caractérisée en ce que** le verrou (12) présente un crochet (10) sur un côté d'une ouverture (7) de la bague (5), qui peut être accroché dans une pièce complémentaire (11) sur un autre côté de l'ouverture (7) de la bague (5).

5. Prothèse d'osselets réglable en longueur selon la revendication 4, **caractérisée en ce que** le verrou (12) présente deux crochets (10) ou plus l'un après l'autre sur un côté d'une ouverture (7) le long de la bague (5), chacun des crochets pouvant être accroché dans la pièce complémentaire (11) sur l'autre côté de l'ouverture (7) de la bague (5), de telle sorte qu'une périphérie de la bague (5) et/ou une distance entre les éléments de guidage et de serrage (8) peuvent être réglées pour s'adapter à des éléments de liaison (4) de différents diamètres.

6. Prothèse d'osselets réglable en longueur selon une ou plusieurs des revendications 3 à 5, **caractérisée en ce que** la bague (5) présente un affaiblissement de section transversale (6) en un emplacement périphérique afin de pouvoir être comprimée plus facilement pour tendre les éléments de guidage et de serrage (8) contre l'élément de liaison (4).

7. Prothèse d'osselets réglable en longueur selon une ou plusieurs des revendications 3 à 6, **caractérisée en ce que** la bague (5) présente, de part et d'autre de l'ouverture (7), des surfaces d'application (13) pour l'application d'un outil de fermeture pour fermer la bague (5) sur son côté extérieur, qui sont formées de telle sorte que l'outil de fermeture appliqué ne glisse pas dans la direction périphérique de la bague (5) lorsqu'il exerce une force de fermeture sur la bague (5).

8. Prothèse d'osselets réglable en longueur selon une ou plusieurs des revendications 3 à 7, **caractérisée en ce que** les éléments de guidage et de serrage (8) sont agencés à une distance plus grande de l'ouverture (7) de la bague (5) que d'un emplacement périphérique opposé à l'ouverture (7).

9. Prothèse d'osselets réglable en longueur selon une ou plusieurs des revendications 1 à 8, **caractérisée en ce que** l'élément de pied (3) est configuré pour être fixé à l'étrier ou à la fenêtre ovale de l'oreille moyenne d'un être humain.
